(19) Europäisches Patentamt
European Patent Office
Office européen des brevets

(11) Publication number: **0 176 151**
**B1**

(12) # EUROPEAN PATENT SPECIFICATION

(45) Date of publication of patent specification: **20.01.88**

(51) Int. Cl.⁴: **C 11 D 1/52,** A 61 K 7/50, C 07 C 103/38

(21) Application number: **85201505.6**

(22) Date of filing: **19.09.85**

(54) **Detergents containing amides as thickening agents.**

(30) Priority: **21.09.84 NL 8402893**

(43) Date of publication of application:
**02.04.86 Bulletin 86/14**

(45) Publication of the grant of the patent:
**20.01.88 Bulletin 88/03**

(84) Designated Contracting States:
**AT BE CH DE FR GB IT LI LU NL SE**

(56) References cited:
**EP-A-0 112 046**
**CH-A- 411 221**
**US-A-3 954 676**

(73) Proprietor: **Chemische Fabrik, Chem-Y, GmbH**
**Kupferstrasse 1**
**D-4240 Emmerich 1 (DE)**

(72) Inventor: **Smid, Jacob Kornelis**
**J.G. Heuthorstraat 61**
**Doetinchem (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 176 151 B1**

**Description**

This invention relates to the use of certain specific amides as thickening agents in detergents which contain certain surface-active substances.

For liquid detergent products which frequently or for a long time are contacted with human hair or human skin, such as shampoos, shower and bath compositions and compositions for non-automatic dishwashing, a certain "body" is desired, i.e. that they have to be thickened. For this purpose various kinds of thickening agents are known and commercially available. Thus, common salt is a known thickening agent, but its drawback is that too high concentrations thereof in cosmetic products can cause eye irritations. Furthermore, the surface-active ether carboxylic acids cannot be thickened with common salt at all. Until recently diethanol amides also were used as thickening agents on a large scale. However, according to some recent theories diethanol amides could form nitrosamines, which can cause cancers, as is well known. Therefore, the market for these products has decreased already markedly.

Another known thickening agent is the distearate of polyethylene glycol 6000. However, this product has a high melting point (55 to 60°C) and the use of more than 1% in a detergent composition causes a slimy feel. Also the product is relatively expensive. A related product is a glucamate having 2 oleyl residues and 2 polyoxyethylene residues having a total of 120 oxyethylene units, which also is a solid. This product does not have a better thickening action than the above mentioned distearate and is much more expensive.

Carboxymethyl cellulose also is a known thickening agent, but the viscosity obtained with this agent is not stable, but decreases with time.

If possible, it is desirable to use a combination of the cheap NaCl with another thickening agent.

Consequently, there is a need of a new thickening agent, particularly for compositions which contain polyether carboxylic acid and/or polyether sulphate as an active detergent. As mentioned already, polyether carboxylic acid cannot be thickened with the cheap NaCl, and in the case of polyether sulphate it is desirable for cosmetic compositions and the like to restrict the amount of NaCl in order to prevent irritation phenomena.

Surprisingly, it has now been found that these objects can be attained with very specific compounds. These compounds are amides of the formula

$$RO{-}(CH_2CH_2O)_nCH_2CO{-}NR'R'',$$

wherein R represents an aliphatic hydrocarbon residue having 8—16 carbon atoms, n is a number having an average value of no more than 5 and the group NR'R'' represents the residue of monoethanol amine, diethanol amine or monoisopropanol amine.

Accordingly, these compounds are substituted amides of a sub group of the ether carboxylic acids. Not only these compounds appear to have a good thickening action on the ether carboxylic acids and also on the ether sulphates, for which purpose they can be easily combined with NaCl, but moreover they are derivatives of the ether carboxylic acids and as such they are surface active themselves and consequently also contribute to the total activity of the composition in this respect.

The present compounds are known as a class from Swiss patent 411221, wherein they have been disclosed as additives for the so-called dye-spinning of regenerated cellulose fibres. There is no suggestion therein of the present use.

Inasmuch as the present additives are monoethanol amides or mono-isopropanol amides, respectively, they cannot give rise to the forming of nitrosamines. The diethanol amides also possess a thickening activity and in this respect they are equivalent or superior to the known products of this type.

The fact that the present amides have the desired thickening activity is particularly surprising, because salts of the ether carboxylic acids with the corresponding amines (monoethanol amine, and monoisopropanolamine, respectively) do not show this action.

The hydrophobic residue of the ether carboxylic acids generally can be a high alkyl or alkylphenyl residue. Because it concerns here compositions intended for prolonged or frequent contact with human hair or human skin, the compounds having an aliphatic residue enter into consideration in the first place for the present thickening agents. It has appeared in this respect that the residue R should not be too high; experiments with a stearyl compound showed an insufficient thickening action. In shampoos and the like often ether carboxylic acids are used derived from alcohols of 12—14 carbon atoms and these residues in general also are very satisfactory for the present thickening agents. Furthermore, the number of epoxy units should not be too high. At values of n higher than 5 the thickening activity is lost. On the other hand, n may also amount to 0. The optimum value for n of course also depends on the exact meaning of R and the choice of the group NR'R''.

The present compounds can be prepared in a usual manner, as mentioend in Swiss patent 411221, starting from the corresponding ether carboxylic acids. A suitable method of preparation is heating of the free acid with the suitable amine with distilling off of water and this preparation offers no problems whatsoever.

As is known in the field of ether carboxylic acids, the number of ethoxy groups usually is an average which is attained by reacting the alcohol ROH with the number of molecules of ethylene oxide which corresponds to the desired average value of n. This reaction can be catalyzed by a base or by a Lewis acid.

In the first case an ethoxylation product is formed having a broad distribution of the values of n and in the case of a low value of n also a large amount of non-ethoxylated product is present. In the case of catalysis with a Lewis acid a much narrower distribution is obtained and with the present thickening agents a narrow distribution gives a somewhat better activity than is the case with the broader distribution. Preferably, the average value of n is 0.5—1.5.

Consequently, the invention provides a detergent composition intended for prolonged or frequent contact with human hair or human skin, said composition containing one or more active detergents of the ether sulphate and/or ether carboxylic acid type, as well as a thickening agent and, if desired, further usual additives, said composition being characterized by the fact that the thickening agent is an amide according to the above definitions.

The invention is further elucidated with the aid of the following experiments.

A standard recipe was used, i.e. as follows:

| | |
|---|---|
| Ether sulphate, $RO(C_2H_4O)_2SO_4Na$ derived from Dobanol 23 ($C_{12}$—$C_{13}$ alcohol mixture, commercial product of Shell), as 28% aqueous solution | 27% |
| Ether carboxylic acid $RO(C_2H_4O)_{10}CH_2COOH$ derived from lauryl and myristyl alcohols (70:30), as 22% aqueous solution, neutralized to pH 7 | 12% |
| Benzylformal (preservative) | 0.07% |

To this were added the thickening agent to be tested, and NaCl and water to 100%.

The two surfactant agents are commercial products of the present applicant.

In a first series of tests 1.75% of NaCl was incorporated in the above mentioned standard recipe, as well as varying percentages of thickening agents. The following products were compared:

z, a commercial product, a diethanol amide of coco fatty acid, prepared by direct amidation of coco oil so that the product also contains glycerol.

a. Lauryl-myristyl (70—30)—O—$(C_2H_4O)_{2.5}CH_2CONH$—$C_2H_4OH$ (narrow distribution of ethoxy groups)

b. Lauryl-myristyl (70:30)—O—$(C_2H_4O)_{4.5}$—$CH_2CONH$—$C_3H_4OH$ (no narrow distribution)

c. Stearyl-O—$(C_2H_4O)_2$—CO—NH—$C_2H_4OH$ (no narrow distribution)

d. Ester of lauryl-myristyl (70:30)—O—$(C_2H_4O)_{4.5}$—$CH_2COOH$ and lauryl-myristyl (70:30)—$O(C_2H_4O)_{4.5}H$ (no narrow distribution).

e. Ester of lauryl-myristyl (70:30)—O—$(C_2H_4O)_{2.5}$—$CH_2COOH$ and lauryl-myristyl (70:30)—O—$C_2H_4O)_{2.5}$—H (both narrow distribution).

f. Monoethanol amine salt of stearyl-O—$(C_2H_4O)_4$—$CH_2COOH$ (narrow distribution).

g. Monoethanol amine salt of lauryl-myristyl (70:30)—O—$(C_2H_4O)_{1.5}$—$CH_2$—COOH (narrow distribution).

TABLE A

Viscosity in mPa.s after addition of varying percentages of thickening agent to standard recipe with 1.75% NaCl.

| Thickening Agent | 1% | 3% | 5% |
|---|---|---|---|
| Product z | 14 | 80 | 1.520 |
| a | 12 | 40 | 740 |
| b | 11 | 12.5 | 20 |
| c | 10 | 10 | 10 |
| d | 12.5 | 45 | 420 |
| e | 15 | —[1] | —[1] |
| f | 10 | 10 | 10 |
| g | 12.5 | 14 | 35 |

[1] Product is insufficiently soluble.

It follows from the above data that product a has a distinct thickening effect.

For product b the thickening effect is small, which presumably is caused by the fact that with respect to this hydrophobic group an average number of 4.5 epoxy groups is already to be considered relatively high. Product c, derived from stearyl alcohol, appears to show no activity. Product d shows a distinct activity, but as has appeared later on, the thickening activity decreases rather quickly, which is caused by a gradual hydrolysis of the ester. Product e appears to be unsuitable because of insufficient solubility. Finally, it appears that the monoethanol amine salts, products f and g, show no or almost no thickening effect.

Subsequently, the response to NaCl addition was investigated for a number of products. In general, when NaCl is added to a composition which contains another thickening agent, a viscosity increase is obtained up to a maximum, whereafter on further addition of NaCl the viscosity decreases again. In the present tests one started from the standard recipe with 2% of thickening agent and varying amounts of NaCl. The results have been rendered in table B.

TABLE B

Viscosity in mPa.s Thickening agent (2%)

| %NaCl | Product z | a | e | f | g |
|---|---|---|---|---|---|
| 0 | 10 | 10 | 10 | 10 | 10 |
| 2 | 40 | 12.5 | 160 | 16 | 15 |
| 3 | 170 | 70 | 1,900 | 25 | 37.5 |
| 4 | 760 | 520 | 400[1)] | 55 | 260 |
| 5 | 1,600 | 1,800 | 300[1)] | 480 | 1,600 |
| 6 | 1,700 | 2,600 | — | 1,850 | 3,200 |
| 7 | 1,300 | 3,200 | — | 4,600 | 6,500 |
| 8 | 800 | 3,700 | — | 8,750 | 5,400 |
| 9 | 300 | 3,300 | — | 11,200 | 1,500 |
| 10 | 200 | 1,900 | — | 9,400 | 600 |

[1)]) The mixture becomes turbid on addition of 4% NaCl and the viscosity then drops.

It appears from these data that product a in combination with NaCl shows a higher viscosity maximum than the product z.

Product e again appears to have a low compatibility and the products f and g indeed yield high maxima, but a reasonable thickening only starts to occur at high salt percentages.

Because it was supposed that modification of product a by lowering the average number of oxyethylene units perhaps would lead to a better effect, a corresponding product was prepared having an average of 1.5 oxyethylene groups (narrow distribution). Furthermore, also another stearyl derivative was tested, this time having a higher average number of oxyethylene units. Accordingly, these are the following products.

h. Lauryl-myristyl (70:30)—O—$(C_2H_4O)_{1.5}$—CONH—$C_2H_4OH$ (narrow distribution)

i. Stearyl-O—$(C_2H_4O)_4$—CONH—$C_2H_4OH$ (narrow distribution).

These two products were compared with product z in the standard recipe with 1.75% NaCl. The results are as follows:

TABLE C

Viscosity in mPa.s (1.75% NaCl)

| | % Thickening agent | | | |
|---|---|---|---|---|
| | 0 | 1 | 3 | 5 |
| Product z | 10 | 14 | 80 | 1,520 |
| h | 10 | 15 | 400 | 27,500 |
| i | 10 | 10 | 10 | 13 |

It follows from these data that product h according to expectation worked even better than product a and that product i practically had no thickening activity so that apparently compounds having a stearyl residue are not suitable.

Subsequently, the effect of increasing amounts of NaCl in the standard recipe with 2% thickening agent was compared for product h and product z. The results are as follows:

TABLE D

Viscosity in mPa.s (2% thickening agent)

| % NaCl | Product z | Product h |
|---|---|---|
| 0 | 10 | 11 |
| 2 | 40 | 50 |
| 3 | 170 | 560 |
| 4 | 760 | 3,100 |
| 5 | 1,600 | 6,200 |
| 6 | 1,700 | 8,000 |
| 7 | 1,300 | 2,500 |

Consequently, product h appears to be clearly superior to the commercial product.

A corresponding test was carried out, but this time with 3% of thickening agent. The results are as follows:

TABLE E

Viscosity in mPa.s (3% thickening agent)

| % NaCl | Product z | Product h |
|---|---|---|
| 0 | 5 | 11 |
| 1 | 8 | 25 |
| 2 | 135 | 450 |
| 3 | 1,080 | 5,700 |
| 4 | 2,100 | 8,200 |
| 5 | 3,000 | 1,750 |
| 6 | 2,100 | 480 |

**Claims for the Contracting States: BE CH DE FR GB IT LI LU NL SE**

1. Detergent compositions intended for prolonged or frequent contact with human hair or human skin, which composition contains one or more active detergents of the ether sulphate and/or ether carboxylic acid type, as well as a thickening agent and, if desired, further usual additives, characterized by the fact that the thickening agent is an amide of the formula

$$RO—(CH_2CH_2O)_nCH_2CO—NR'R'',$$

wherein R is an aliphatic hydrocarbon residue of 8—16 carbon atoms, n is a number having an average value of no more than 5 and the group NR'R'' represents the residue of monoethanol amine, diethanol amine or monoisopropanol amine.

2. Detergent composition according to claim 1, characterized by the fact that the thickening agent is an amide according to claim 1, wherein R is derived from natural fatty alcohols of 12—14 carbon atoms.

3. Thickening agent according to claim 1 or 2, characterized by the fact that the thickener is an amide of the formula given in claim 1, wherein n has an average value of 0.5—1.5.

4. Use of amides of the formula

$$RO—(CH_2CH_2O)_nCH_2CO—NR'R'',$$

wherein R represents an aliphatic hydrocarbon residue of 8—16 carbon atoms, n is a number having an average value of no more than 5 and the group NR'R'' represents the residue of monoethanol amine, diethanol amine or monoisopropanol amine, for thickening detergent compositions which contain one or more active detergents of the ether sulphate and/or ether carboxylic acid type.

**Claims for the Contracting State: AT**

1. A process for preparing a detergent composition intended for prolonged or frequent contact with human hair or human skin, by incorporating into this composition one or more active detergents of the ether sulphate and/or ether carboxylic acid type, as well as a thickening agent and, if desired, further usual additives, characterized by using as the thickening agent an amide of the formula

$$RO—(CH_2CH_2O)_nCH_2CO—NR'R'',$$

wherein R is an aliphatic hydrocarbon residue of 8—16 carbon atoms, n is a number having an average value of no more than 5 and the group NR'R'' represents the residue of monoethanol amine, diethanol amine or monoisopropanol amine.

2. A process according to claim 1, characterized by using an amide wherein R is derived from natural fatty alcohols of 12—14 carbon atoms.

3. A process according to claim 1 or 2, characterized by using an amide wherein n has an average value of 0.5—1.5.

4. Use of amides of the formula

$$RO—(CH_2CH_2O)_nCH_2CO—NR'R'',$$

wherein R is an aliphatic hydrocarbon residue of 8—16 carbon atoms, n is a number having an average value of no more than 5 and the group NR'R'' represents the residue of monoethanol amine, diethanol amine or monoisopropanol amine, for thickening detergent compositions which contain one or more active detergents of the ether sulphate and/or ether carboxylic acid type.

**Patentansprüche für die Vertragsstaaten: BE CH DE FR GB IT LI LU NL SE**

1. Reinigungsmittel, das für den längeren oder häufigen Kontakt mit menschlichem Haar oder menschlicher Haut bestimmt ist, enthaltend eine oder mehrere aktive Reinigungssubstanzen von der Art der Ethersulfate und/oder Ethercarbonsäuren sowie ein Verdickungsmittel und, gegebenenfalls, weitere übliche Zusätze, dadurch gekennzeichnet, daß das Verdickungsmittel ein Amid der Formel

$$RO—(CH_2CH_2O)_nCH_2CO—NR'R'',$$

worin R ein aliphatischer Kohlenwasserstoff mit 8—16 Kohlenstoffatomen und n eine Zahl mit einem Durchschnittswert von nicht mehr als 5 ist und die Gruppe NR'R'' den Monoethanolamin-, Diethanolamin- oder Monoisopropanolaminrest darstellt, ist.

2. Reinigungsmittel nach Anspruch 1, dadurch gekennzeichnet, daß das Verdickungsmittel ein Amid gemäß Anspruch 1 ist, in welchem R von natürlichen Fettalkoholen mit 12—14 Kohlenstoffatomen abgeleitet ist.

3. Reinigungsmittel nach Anspruch 1 oder 2, dadurch gekennzeichnet, daß das Verdickungsmittel ein Amid der in Anspruch 1 angegebenen Formel ist, worin n einen Durchschnittswert von 0,5—1,5 hat.

4. Verwendung von Amiden der Formel

$$RO—(CH_2CH_2O)_nCH_2CO—NR'R'',$$

worin R ein aliphatischer Kohlenwasserstoffrest mit 8—16 Kohlenstoffatomen und n eine Zahl mit einem Durchschnittswert von nicht mehr als 5 ist und die Gruppe NR'R'' den Monoethanolamin-, Diethanolamin- oder Monoisopropanolaminrest darstellt, zur Verdickung von Reinigungsmitteln, die eine oder mehrere aktive Reinigungssubstanzen von der Art der Ethersulfate und/oder Ethercarbonsäuren enthalten.

**Patentansprüche für den Vertragsstat: AT**

1. Verfahren zur Herstellung eines Reinigungsmittels, das für den längeren oder häufigen Kontakt mit menschlichem Haar oder menschlicher Haut bestimmt ist, indem man in dieses Mittel ein oder mehrere aktive Reinigungssubstanzen von der Art der Ethersulfate und/oder Ethercarbonsäuren sowie ein Verdickungsmittel und, gegebenenfalls, weitere übliche Zusätze inkorporiert, dadurch gekennzeichnet, daß als Verdickungsmittel ein Amid der Formel

$$RO—(CH_2CH_2O)_nCH_2CO—NR'R'',$$

worin R ein aliphatischer Kohlenwasserstoff mit 8—16 Kohlenstoffatomen und n eine Zahl mit einem Durchschnittswert von nicht mehr als 5 ist und die Gruppe NR'R'' den Monoethanolamin-, Diethanolamin- oder Monoisopropanolaminrest darstellt, verwendet wird.

2. Verfahren nach Anspruch 1, dadurch gekennzeichnet, daß ein Amid verwendet wird, in welchem R von natürlichen Fettalkoholen mit 12—14 Kohlenstoffatomen abgeleitet ist.

3. Verfahren nach Anspruch 1 oder 2, dadurch gekennzeichnet, daß ein Amid verwendet wird, in welchem n einen Durchschnittswert von 0,5—1,5 hat.

4. Verwendung von Amiden der Formel

$$RO—(CH_2CH_2O)_nCH_2CO—NR'R'',$$

worin R ein aliphatischer Kohlenwasserstoffrest mit 8—16 Kohlenstoffatomen und n eine Zahl mit einem Durchschnittswert von nicht mehr als 5 ist und die Gruppe NR'R'' den Monoethanolamin-, Diethanolamin- oder Monoisopropanolaminrest darstellt, zur Verdickung von Reinigungsmitteln, die eine oder mehrere aktive Reinigungssubstanzen von der Art der Ethersulfate und/oder Ethercarbonsäuren enthalten.

**Revendications pour les Etats contractants: BE CH DE FR GB IT LI LU NL SE**

1. Composition détergente destinée à un contact prolongé ou fréquent avec les cheveux humains ou la peau humaine, cette composition contenant un ou plusieurs détergents actifs du type éther-sulfate et/ou acide éther-carboxylique, ainsi qu'un agent épaississant et, éventuellement, d'autres additifs habituels, caractérisée par le fait que l'agent épaississant est un amide de formule

$$RO—(CH_2CH_2O)_nCH_2CO—NR'R'',$$

dans laquelle R est un résidu hydrocarboné aliphatique ayant 8 à 16 atomes de carbone, n est un nombre ayant une valeur moyenne au plus égale à 5 et le groupe NR'R'' représente le résidu monoéthanolamine, diéthanolamine ou monoisopropanolamine.

2. Composition détergente selon la revendication 1, caractérisée par le fait que l'agent épaississant est un amide selon la revendication 1, dans laquelle R est dérivé d'alcools gras naturels ayant 12 à 14 atomes de carbone.

3. Agent épaississant selon la revendication 1 ou 2, caractérisé par le fait que l'épaississant est un amide de formule donnée dans la revendication 1, dans laquelle n a une valeur moyenne de 0,5—1,5.

4. Utilisation des amides de formule

$$RO—(CH_2CH_2O)_nCH_2CO—NR'R'',$$

dans laquelle R représente un résidu hydrocarboné aliphatique ayant 8 à 16 atomes de carbone, n est un nombre ayant une valeur moyenne au plus égale à 5 et le groupe NR'R'' représente le résidu monoéthanolamine, diéthanolamine ou monoisopropanolamine, pour des compositions détergentes épaississantes qui contiennent un ou plusieurs détergents actifs du type éther-sulfate et/ou acide éther-carboxylique.

**Revendications pour l'Etat contractant: AT**

1. Procédé de préparation d'une composition détergente destinée à un contact prolongé ou fréquent avec les cheveux humains ou la peau humaine, qui consiste à incorporer, dans cette composition, un ou plusieurs détergents actifs du type éther-sulfate et/ou acide éther-carboxylique, ainsi qu'un agent épaississant et, éventuellement, d'autres additifs habituels, caractérisé en ce qu'on utilise, comme agent épaississant, un amide de formule

$$RO—(CH_2CH_2O)_nCH_2CO—NR'R'',$$

dans laquelle R est un résidu hydrocarboné aliphatique ayant 8 à 16 atomes de carbone, n est un nombre ayant une valeur moyenne au plus égale à 5 et le groupe NR'R'' représente le résidu monoéthanolamine, diéthanolamine ou monoisopropanolamine.

2. Procédé selon la revendication 1, caractérisé en ce qu'on utilise un amide, dans lequel R est dérivé d'alcools gras naturels ayant 12 à 14 atomes de carbone.

3. Procédé selon la revendication 1 ou 2, caractérisé en ce qu'on utilise un amide, dans lequel n a une valeur moyenne de 0,5—1,5.

4. Utilisation des amides de formule

$$RO—(CH_2CH_2O)_nCH_2CO—NR'R'',$$

dans laquelle R représente un résidu hydrocarboné aliphatique ayant 8 à 16 atomes de carbone, n est un nombre ayant une valeur moyenne au plus égale à 5 et le groupe NR'R'' représente le résidu monoéthanolamine, diéthanolamine ou monoisopropanolamine, pour des compositions détergentes épaississantes qui contiennent un ou plusieurs détergents actifs du type éther-sulfate et/ou acide éther-carboxylique.